# EUROPÄISCHE PATENTSCHRIFT

(11) **EP 1 462 160 B1**
(45) Veröffentlichungstag und Bekanntmachung des Hinweises auf die Patenterteilung: **29.08.2007**
(21) Anmeldenummer: 04007198.7
(22) Anmeldetag: 25.03.2004
(51) Int. Cl.: B01J 19/02, C07C 2/00, B01J 19/00, B01J 12/00

(54) **Verfahren zur Durchführung einer Hochtemperaturreaktion, Reaktor zur Durchführung des Verfahrens, sowie Verwendung**
Process to obtain a high-temperature reaction, reactor for the implementation of this process and use thereof
Procédé pour la réalisation d' une réaction à haute température , réacteur pour la mise en oeuvre de ce procédé et son utilisation

(30) Priorität: 26.03.2003 DE 10313527
(43) Veröffentlichungstag der Anmeldung: 29.09.2004
(73) Patentinhaber: BASF Aktiengesellschaft, 67056 Ludwigshafen (DE)
(72) Erfinder: Bartenbach, Bernd, Dr., 67117 Limburgerhof (DE); Bachtler, Michael, Dr., 76857 Albersweiler (DE); Schmitz-Bäder, Petra, 67227 Frankenthal (DE); Scheidsteger, Olaf, Dr., 68163 Mannheim (DE); Stapf, Dieter, Dr., 2930 Brasschaat (BE)
(74) Vertreter: Isenbruck, Günter

(56) Entgegenhaltungen:
- WO-A-01/37984
- GB-A- 2 121 313
- US-A- 2 158 582
- US-A- 2 679 543
- US-A- 2 765 358
- US-A- 3 287 434
- US-A- 3 438 741
- US-A- 3 542 894
- US-A- 5 407 455
- US-A- 5 932 182
- US-B1- 6 258 330

## Beschreibung

Die Erfindung betrifft ein Verfahren zur Durchführung einer Hochtemperaturreaktion mit kurzer Verweilzeit, bei der das Reaktionsgemisch anschließend in einem Quenchbereich schnell abgekühlt wird, einen Reaktor zur Durchführung des Verfahrens, sowie eine Verwendung.

Als Hochtemperaturreaktionen werden in der Regel Reaktionen bezeichnet, die bei einer Temperatur oberhalb von 800°C durchgeführt werden. Als kurz werden vorliegend Verweilzeiten im Millisekunden-Bereich, insbesondere im Bereich von etwa 1 bis 100 ms, verstanden. Analog wird als schnelle Abkühlung eine Abkühlung im Millisekunden-Bereich, insbesondere im Bereich von etwa 1 bis 100 ms, verstanden.

Großtechnisch wichtige Hochtemperaturreaktionen mit anschließender schneller Abkühlung sind beispielsweise Reaktionen zur Herstellung von Acetylen. Ein Verfahren zur Herstellung von Acetylen durch partielle Oxidation von Kohlenwasserstoffen mit Sauerstoff wird in der DE-A 44 22 815 beschrieben. Um die zur Reaktion notwendigen hohen Temperaturen zu erreichen, werden die Ausgangsstoffe Erdgas und Sauerstoff möglichst auf bis zu 700°C getrennt vorgeheizt, in einer Mischzone intensiv vermischt und durch einen mit Kanälen versehenen Brennerblock in einem Reaktionsraum zur Reaktion gebracht. Dabei beträgt das Volumenverhältnis von eingesetztem Sauerstoff zu eingesetztem Erdgas etwa 0,6. Die Geschwindigkeit der Sauerstoff/Erdgas-Mischung in den Kanälen des Brennerblocks muss so hoch sein, dass ein Durchschlagen der Flamme in den Mischraum verhindert wird. Der Reaktionsraum, der sich dem Brennerblock anschließt, ist so bemessen, dass die Verweilzeit des acetylenhaltigen Reaktionsgases, des sogenannten Spaltgases, nur wenige Millisekunden beträgt. Nach dieser Zeit, innerhalb der sich die dem Temperaturniveau dieser Reaktion entsprechenden Gleichgewichte nicht einstellen können, werden die Reaktionsprodukte möglichst schnell auf unter 300°C mit Wasser oder einem Rückstandsöl abgeschreckt, damit das gebildete Acetylen nicht in Ruß und Wasserstoff zerfällt. Als nachteilig bei diesem Verfahren zeigt sich, dass die hohe Energie des Spaltgases nicht weiter genutzt werden kann.

Die EP-A 1 041 037 beschreibt ein sogenanntes Niedertemperaturverfahren zur Herstellung von Acetylen und Synthesegas. Dieses Verfahren hat die Besonderheit, dass während des Verfahrens Temperaturen von maximal 1400°C erreicht werden, während die Herstellung von Acetylen, wie sie in der DE-A 44 22 815 beschrieben wird, bei einer Temperatur von mindestens 1500°C abläuft. Durch die verhältnismäßig lange mittlere Verweilzeit im Reaktor - in der Regel mindestens 10 ms - kann das Reaktionsgemisch durch indirekte Kühlung oder durch Kombination aus direktem Quench und indirekter Kühlung abgekühlt werden. Hierdurch wird es möglich, die Reaktionswärme durch Einsatz eines geeigneten Wärmetauschers, zum Beispiel zur Erzeugung von Hochdruckdampf, zu nutzen. Allerdings erfüllen die Ausbeute und die Russbildung beim Niedertemperatur-Acetylenverfahren nicht immer die wirtschaftlichen Anforderungen.

Aus US 2,679,543 ist ein Verfahren zur Herstellung von Acetylen bei einer Reaktionstemperatur von 1100 bis 1500°C und Verweilzeiten im Bereich von 0,001 bis 0,05 Sekunden bekannt, wonach das Reaktionsgemisch nach der Reaktion unmittelbar auf eine Temperatur von höchstens 600 bis 650°C durch direkten Quench und anschließend durch indirekte Kühlung auf etwa 90°C abgekühlt wird. Den Ausführungen in der genannten Druckschrift zufolge ist es für die Energierückgewinnung in der nachfolgenden indirekten Abkühlung wichtig, im ersten Abkühlungsschritt, dem direkten Quench, eine möglichst hohe Maximaltemperatur anzustreben, hierfür dürfe jedoch eine Maximaltemperatur von etwa 600 bis 650°C nicht überschritten werden.

Die US 2,765,358 beschreibt ein Verfahren zur Herstellung von Acetylen durch Oxidation von gasförmigen Kohlenwasserstoffen mit Sauerstoff in einer Reaktionszone bei einer Temperatur oberhalb von etwa 1370 °C, wobei die Reaktanden bei einer Temperatur unterhalb von etwa 315 °C gemischt, mit hoher Geschwindigkeit durch von außen beheizte Rohre mit kleinem Durchmesser geleitet, wobei die Temperatur auf mindestens 600 °C erhöht wird, und anschließend über eine Barriere mit gewundenen Kanälen, aus einem feuerfesten Material in den Reaktionsraum geleitet wird.

Die US 4,288,408 beschreibt einen Hochtemperaturreaktor zum Cracken von Kohlenwasserstoffströmen, wobei die Reaktorwände aus einer Aluminiumoxid-Feuerfestkeramik gebildet sein können.

Es war demgegenüber Aufgabe der Erfindung, ein Verfahren zur Durchführung einer Hochtemperaturreaktion zur Verfügung zu stellen, bei dem die Reaktionswärme weitestgehend genutzt wird und gleichzeitig eine Ausbeute erzielt wird, die wirtschaftlichen Anforderungen genügt.

Die Aufgabe wird gelöst durch ein Verfahren zur Durchführung einer Hochtemperaturreaktion, bei der die Edukte durch Kanäle eines Brennerblocks einem Reaktionsraum zugeführt werden, wobei im Reaktionsraum die Hochtemperaturreaktion mit kurzer Verweilzeit bei einer Temperatur von mindestens 1500°C stattfindet und das Reaktionsgemisch anschließend in einem Quenchbereich schnell abgekühlt wird, das dadurch gekennzeichnet ist, dass im Quenchbereich zunächst eine direkte Abkühlung auf eine Temperatur im Bereich von 650°C bis 1200°C durch Zuführung eines verdampfenden Quenchmediums und anschließend eine indirekte Abkühlung in einem Wärmeübertrager erfolgt. Die Edukte werden vorzugsweise vorvermischt.

Die Hochtemperaturreaktion ist insbesondere eine Reaktion zur Herstellung von Acetylen durch partielle Oxidation von Kohlenwasserstoffen mit Sauerstoff, die vorteilhaft bei einer Temperatur im Bereich von 1550 bis 1750°C durchgeführt wird.

Es wurde überraschend gefunden, dass es möglich ist, eine Hochtemperaturreaktion bei hohen Temperaturen, von mindestens 1500°C, bevorzugt von etwa 1550 bis 1750°C durchzuführen, und dabei das heiße Reaktions-Gas-Gemisch in einem ersten, direkten Teilquench auf eine ebenfalls noch hohe Temperatur im Bereich von 650 bis 1200°C und anschließend indirekt in einem zweiten Teilquench abzukühlen.

Es wurde somit gefunden, dass gegenüber dem aus US 2,679,548 bekannten Verfahren eine wesentlich geringere Abkühlung im ersten Teilquench erforderlich ist und somit wesentlich mehr Reaktionswärme für den zweiten, indirekten Teilquench zur Verfügung steht, die beispielsweise zur Hochdruckdampferzeugung genutzt werden kann. Es war bekannt, dass der Acetylenzerfall kinetisch kontrolliert ist und somit die Abkühlgeschwindigkeit für die Acetylenverluste durch Zersetzung maßgeblich ist. Trotzdem wurde überraschend gefunden, dass die Reaktion auf einem hohen Temperaturniveau durchgeführt werden kann und für die Abkühlung im ersten, direkten Quench relativ hohe Maximaltemperaturen zugelassen werden können ohne negative Auswirkungen auf die Ausbeute.

Aufgrund der immer noch hohen Temperaturen nach dem ersten Quench verdampft das Wasser bzw. Kohlenwasserstoffgemisch, das als Quenchmedium eingesetzt wird, vollständig. Aus diesem Grund wird der erste Quench auch als Trockenquench bezeichnet.

Die indirekte Abkühlung in einem Wärmeübertrager im zweiten Quenchabschnitt kann dazu genutzt werden, Hochdruckdampf zu erzeugen, der zur weiteren Nutzung zur Verfügung gestellt werden kann oder auch um die Edukte für die Reaktion vorzuheizen.

Die schnelle Abkühlung erfolgt im eingangs definierten Millisekunden-Bereich, insbesondere im Bereich von etwa 1 bis 100 ms, besonders bevorzugt 1 bis 50 ms. Die obigen Abkühlzeiten gelten für die Summe aus direkter und indirekter Abkühlung, wobei die direkte Abkühlung vorzugsweise gegenüber der indirekten kürzer ist.

Bevorzugt erfolgt die direkte Abkühlung auf eine Temperatur im Bereich von 700°C bis 1000°C.

Vorteilhaft wird die direkte Abkühlung ein- oder mehrstufig durchgeführt.

Als Quenchmedium für die direkte Abkühlung wird vorteilhaft Wasser oder ein Kohlenwasserstoff oder ein Kohlenwasserstoffgemisch eingesetzt.

Die indirekte Abkühlung erfolgt vorteilhaft auf weniger als 300°C. Bevorzugt wird die indirekte Abkühlung zur Vorheizung der Edukte genutzt.

Alternativ oder zusätzlich ist es möglich, die indirekte Abkühlung zur Dampferzeugung zu nutzen.

Bei der Herstellung von Acetylen bei hohen Temperaturen zerfällt ein Teil des produzierten Acetylens zu Ruß und Wasserstoff. Der Ruß setzt sich, insbesondere während der Bildungsphase aufgrund seiner hohen Oberflächenaktivität durch thermophoretische Vorgänge und Kondensationsvorgänge bevorzugt auf kalten Oberflächen ab. Dieser Effekt ist besonders stark im Bereich von Rückströmzonen, wie sie zum Beispiel an den Zwickelgebieten der Brennerbohrungen auftreten.

Um eine solche Rußabscheidung und damit Koksbildung zu verhindern, können die Wände des Reaktionsraumes mit einer Feuerfestkeramik ausgekleidet werden. Damit die Feuerfestkeramik den Temperaturen der Hochtemperaturreaktion genügt, weist sie einen Aluminiumoxid-Anteil von mindestens 80 Gew.-%, bevorzugt von mindestens 95 Gew.-%, besonders bevorzugt von mindestens 96 Gew.-% auf.

Die Keramik kann entweder in Form von Steinen oder Blöcken, die bereits ausgehärtet und gebrannt sind, in den Reaktionsraum eingebracht werden oder aber als Guss- oder Stampfmasse, die erst im Reaktionsraum verdichtet, getrocknet und gebrannt wird. Hierbei erfolgt der Brennvorgang bevorzugt durch die Hochtemperaturreaktion selbst.

Die so eingebrachte Keramik weist eine Dicke im Bereich von 7 bis 30 cm auf, bevorzugt hat sie eine Dicke von 8 bis 10 cm. Zusätzlich kann eine Hinterisolierung aus einer Keramik mit besonders guten wärmeisolierenden Eigenschaften erfolgen.

Da beim direkten Quenchen das Kühlmedium nach dem Düsen stark auffächert und aufgrund der hohen Temperatur schnell verdampft, kann der Quenchbereich nicht beliebig vergrößert werden. Aufgrund der schnellen Verdampfung des Kühlmediums treten sonst heiße Strähnen und Inhomogenitäten im Quenchbereich auft. Diese Inhomogenitäten und heißen Strähnen führen dazu, dass in den heißen Bereichen das Acetylen zerfällt.

Aus diesem Grund wird der Übergang des Reaktionsraumes in den Quenchbereich in Form eines Spaltes, der eine Breite im Bereich von 2 bis 200 mm aufweist, ausgebildet. Beim Scale-Up für eine Durchsatzvergrößerung kann so der Reaktionsraum vergrößert werden, wobei aber das Maß des Spaltes am Übergang des Reaktionsraumes in den Quenchbereich beizubehalten ist. Gegenstand der Erfindung ist somit auch ein Verfahren zum Scale-up eines Reaktors, wonach man für eine Durchsatzvergrößerung den Innendurchmesser des Reaktors vergrößert und das Spaltmaß am Übergang vom Reaktionsraum in den Quenchbereich konstant hält.

Bevorzugt ist der Übergang vom Reaktionsraum in den Quenchbereich auf einen Spalt mit einer Breite im Bereich von 50 bis 150 mm begrenzt.

Um die Spaltgeometrie auch in bereits vorhandene Anlagen integrieren zu können, wird der Spalt bevorzugt als Ringspalt ausgebildet. Eine gleichmäßige Strömung des Reaktionsgemisches am Übergang vom Reaktionsraum in den Quenchbereich bei ringförmig ausgebildetem Übergang erreicht man am Besten dadurch, indem der Reaktionsraum ebenfalls in Form eines Ringspaltes ausgebildet ist.

Ebenfalls günstig für die Strömungsführung ist es, wenn die Kanäle im Brennerblock in Richtung der Längsachse des Reaktionsraumes ausgerichtet sind.

Neben den in Längsachse des Reaktionsraumes ausgerichteten Kanälen können ein Teil der Kanäle für das Reaktionsgemisch und/oder Kanäle für die Zuführung von zusätzlichem Sauerstoff oder von Reaktionshilfsstoffen in einem beliebigen Winkel zur Längsachse des Reaktionsraumes ausgerichtet sein.

Bevorzugt ist der Quenchbereich fluchtend in Richtung der Längsachse des Reaktionsraumes, insbesondere als Spalt, besonders bevorzugt als Ringspalt, ausgeführt.
Eine gleichmäßige Strömung von der kreisförmigen Geometrie auf die Ringspaltgeometrie wird durch das Anbringen einer Nabenversperrung erreicht. Hierfür weist die Nabenversperrung bevorzugt die Form eines Kegels oder eines Halbellipsoiden auf.

Die Zufuhr des Quenchmediums zur direkten Abkühlung kann ein- oder mehrstufig erfolgen, wofür Quenchdüsen an einem oder mehreren Verteilern, bei einer Ringspaltgeometrie bevorzugt an einem oder mehreren Ringverteilern, angebracht sind. Das Quenchmedium kann dabei dem Quenchbereich von beiden Seiten des Spaltes, das bedeutet bei einem Ringspalt von außen und/oder von innen zugeführt werden.

Die Eindüsung kann bevorzugt senkrecht zur Längsachse des Quenchbereichs erfolgen, wobei, in der Querschnittsebene senkrecht zur Längsrichtung des Quenchbereichs betrachtet, sowohl eine radiale als auch eine tangentiale Orientierung möglich ist. Die Eindüsung kann auch in einem Winkel zur Längsachse des Quenchbereichs erfolgen.

Bei mehrstufigem Zuführen bei tangentialer Anordnung ist dabei eine gegenläufige Anstellung der Quenchdüsen bevorzugt.

Gegenstand der Erfindung ist auch die Verwendung des vorstehend beschriebenen Verfahrens oder des vorstehend beschriebenen Reaktors zur Herstellung von Acetylen durch partielle Oxidation von Kohlenwasserstoffen mit Sauerstoff.

Im Folgenden wird die Erfindung anhand einer Zeichnung und eines Ausführungsbeispiels näher erläutert.

Es zeigt:
- Figur 1: eine Ausführungsform eines erfindungsgemäßen Reaktors zur Acetylenherstellung mit ringspaltförmig ausgebildetem Reaktionsraum und mit erstem und zweitem Teilquench,
- Figur 2: einen ringspaltförmig ausgebildeten Reaktionsraum mit Brennerblock und direktem Quench,
- Figur 3: einen Schnitt durch einen ringspaltförmig ausgebildeten Brennerblock.

Im Folgenden bezeichnen gleiche Bezugszeichen gleiche oder entsprechende Merkmale.

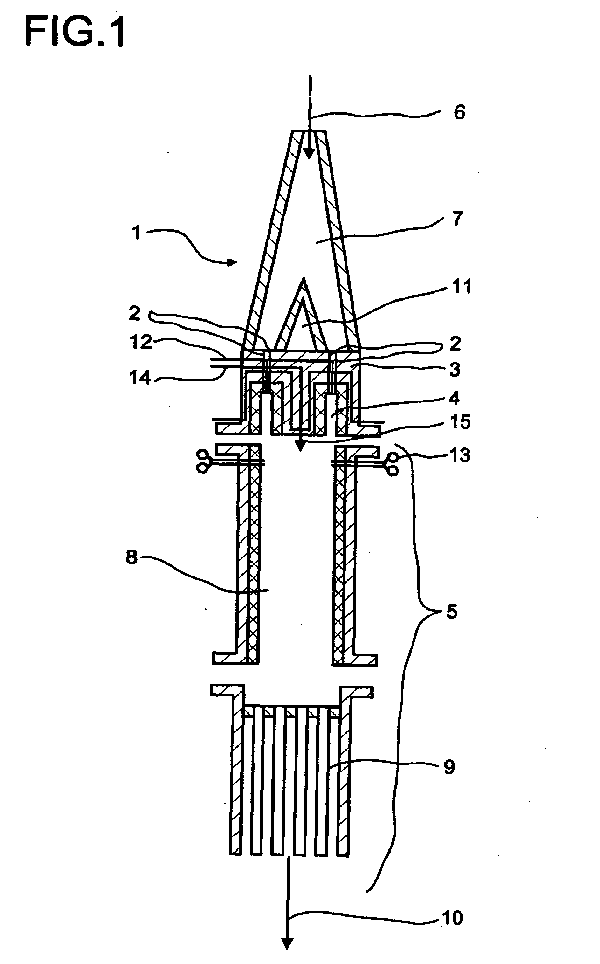

Figur 1 ist ein erfindungsgemäß ausgebildeter Reaktor zur Acetylenherstellung mit erstem und zweitem Teilquench entnehmbar.

Einem Reaktor 1 zur Acetylenherstellung werden als Edukte Sauerstoff oder ein sauerstoffhaltiges Gas und ein Kohlenwasserstoff oder Kohlenwasserstoffgemisch, die jeweils vorgeheizt und vorvermischt werden, über eine Aufgabestelle 6 zugeführt. Zur Vermeidung von Strömungsablösungen und Rückströmungen wird das Gemisch aus Sauerstoff oder sauerstoffhaltigem Gas und Kohlenwasserstoff oder Kohlenwasserstoffgemisch über einen Diffusor 7 und einen mit Kanälen 2 versehenen Brennerblock 3 einem Reaktionsraum 4 zugeführt. Bei einem ringspaltförmig ausgebildeten Reaktionsraum 4 befindet sich im Diffusor 7 eine Nabenversperrung 11, die so ausgebildet ist, dass Rückströmungen und Strömungsablösungen vermieden werden. Die bevorzugte Geometrie für die Nabenversperrung 11 ist eine Kegelform bzw. die Form eines Halbellipsoiden. Im Reaktionsraum 4 wird das Reaktionsgemisch zur Reaktion gebracht. Um einen Rückschlag der dabei entstehenden Flamme in den Brennerblock 3 bzw. Diffusor 7 zu vermeiden und um eine kurze Verweilzeit des Reaktionsgemisches im Reaktionsraum 4 zu gewährleisten, strömt das Reaktionsgemisch mit einer hohen Geschwindigkeit. Nach der Reaktion im Reaktionsraum 4 gelangt das Reaktionsgemisch zur Abkühlung in einen Quenchbereich 5. Hier erfolgt zunächst in einem ersten Teilquench 8 eine direkte Abkühlung auf eine Temperatur im Bereich zwischen 650 und 1200°C, bevorzugt auf eine Temperatur im Bereich von 700°C bis 1000°C. Für die direkte Abkühlung im ersten Teilquench 8 wird das Quenchmedium über Ringverteiler 13 über äußere Quenchdüsen oder über eine Leitung 14 und innere Quenchdüsen 15 in den ersten Teilquench 8 eingedüst. Nach dem ersten Teilquench 8 wird das Reaktionsgemisch in einem zweiten Teilquench 9 weiter auf eine Temperatur im Bereich von 100°C bis 300°C abgekühlt. Hierbei kann die Abkühlung im zweiten Teilquench durch einen indirekten Wärmetausch erfolgen. Der hierfür eingesetzte Wärmeübertrager kann zum Beispiel zur Erzeugung von Hochdruckdampf oder zur Vorheizung der Edukte genutzt werden. Insgesamt ist darauf zu achten, dass die Abkühlphase eine Zeit von 100 ms nicht überschreitet. Um dies zu erreichen, muss die Geschwindigkeit der Reaktionsprodukte ausreichend hoch gewählt werden.

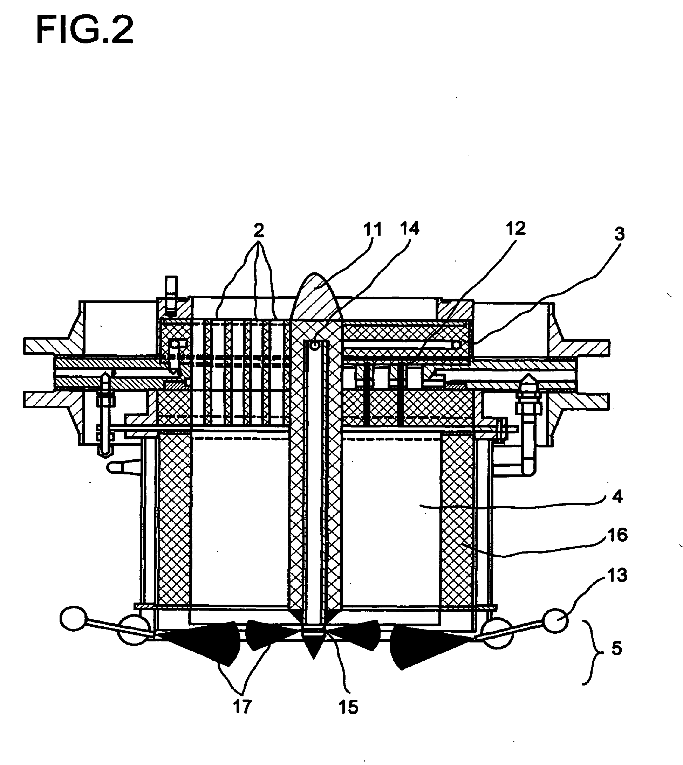

Figur 2 zeigt einen Ausschnitt aus einem Reaktor 1 zur Acetylenherstellung, der den Brennerblock 3 mit Kanälen 2 für die Zufuhr des Reaktionsgemisches und zusätzlichen Kanälen 12 für die Zufuhr von Reaktionshilfsstoffen oder zusätzlichem Sauerstoff, den Reaktionsraum 4 und den direkten Quenchbereich im ersten Teilquench 8 umfasst.

Zur Vermeidung von Ruß oder Koksanbackungen sind die Wände des Reaktionsraumes 4 mit einer Feuerfestkeramik 16 ausgekleidet. Die Nabenversperrung 11, durch die verhindert wird, dass das zugeführte Reaktionsgemisch rückströmt oder Verwirbelungen bildet, ist hier in Form eines Halbellipsoiden ausgebildet. Das Quenchmedium zur direkten Abkühlung wird für das Einsprühen von außen über den Quenchverteiler 13 und für das Einsprühen von innen über die Leitung 14 dem ersten Teilquench 8 zugeführt. Das Quenchmedium verlässt die äußeren Quenchdüsen 15.1 und die inneren Quenchdüsen 15.2 in Form eines Sprühstrahls 17. Die Menge des Quenchmediums ist so eingestellt, dass das Quenchmedium im Sprühstrahl 17 vollständig verdampft, damit kein flüssiges Quenchmedium mehr mitgerissen wird und die Temperatur nach dem ersten Teilquench 8 im Bereich von 600°C bis 1200°C verbleibt.

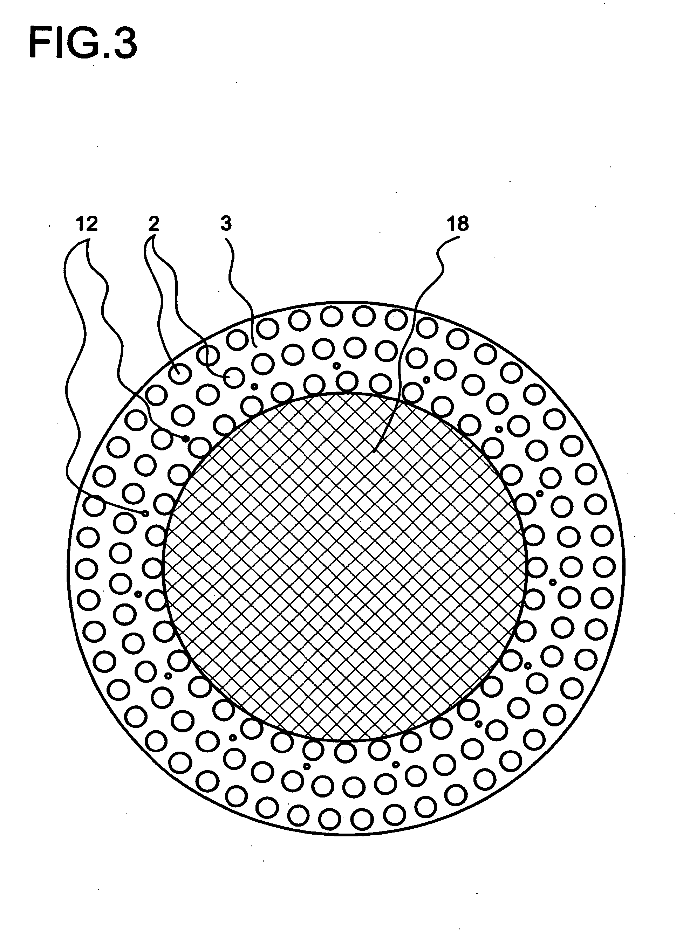

Figur 3 zeigt einen Querschnitt durch einen Brennerblock 3, wie er für den ringspaltförmigen Reaktionsraum 4 eingesetzt wird. Der Brennerblock 3 weist konzentrisch angeordnet die Kanäle 2 für die Zufuhr des Reaktionsgemisches in den Reaktionsraum 4 auf. Weiterhin sind konzentrisch Kanäle 12 für die Zufuhr von zusätzlichem Sauerstoff oder Reaktionshilfsstoffen angebracht. Der innere Bereich 18 des ringförmig ausgebildeten Brennerblocks 3 ist aus einer Feuerfestkeramik ausgebildet.

### Ausführungsbeispiel

Die Funktion des erfindungsgemäßen Verfahrens zur Herstellung von Acetylen und Synthesegas mit einem ersten und zweiten Teilquench wurde an einer modifizierten Anlage untersucht. Hierzu wurden Sauerstoff und Erdgas jeweils auf 600°C vorgewärmt und in einem volumetrischen Verhältnis von 0,59 kontinuierlich gemischt und zur Reaktion gebracht. Die sich ausbildende Flamme wurde kurz nach der Hauptreaktionszone durch Eindüsung von Wasser über einen konzentrischen Kranz von Düsen in ca. 10 bis 50 ms auf eine Temperatur von 850°C abgekühlt. Dabei verdampfte das eingedüste Wasser vollständig. Nach der Eindüsung des Wassers wurde das Produktgasgemisch über einen indirekten Wärmetauscher unter Erzeugung von Hochdruckdampf auf ca. 200°C abgekühlt. Das Produktgasgemisch enthielt nach dem Auskondensieren des Wassers 7,9% Acetylen, 3,4% Kohlendioxid, 5,5% Methan, 25,2% Kohlenmonoxid und 56,4% Wasserstoff. Damit betrug die Ausbeute ca. 29% auf Kohlenstoff bezogen. Zum Vergleich kann das in der Produktion eingesetzte Sachsse-Bartholomé-Verfahren herangezogen werden, bei dem unter gleichen Randbedingungen eine Ausbeute von 29,5% Acetylen, bezogen auf Kohlenstoff erreicht wird.

### Bezugszeichenliste

- 1.: Reaktor
- 2.: Kanäle
- 3.: Brennerblock
- 4.: Reaktionsraum
- 5.: Quenchbereich
- 6.: Aufgabestelle
- 7.: Diffusor
- 8.: erster Teilquench
- 9.: zweiter Teilquench
- 10.: Ablauf
- 11.: Nabenversperrung
- 12.: Zusatzkanäle
- 13.: Quenchverteiler
- 14.: Leitung
- 15.1: äußere Quenchdüsen
- 15.2: innere Quenchdüsen
- 16.: Feuerfestkeramik
- 17.: Sprühstrahl
- 18.: innerer Bereich

## Patentansprüche

1. Verfahren zur Durchführung einer Hochtemperaturreaktion, bei der Edukte durch Kanäle (2) eines Brennerblocks (3) einem Reaktionsraum (4) zugeführt werden, wobei im Reaktionsraum (4) die Hochtemperaturreaktion mit kurzer Verweilzeit bei einer Temperatur von mindestens 1500°C stattfindet und das Reaktionsgemisch anschließend in einem Quenchbereich (5) schnell abgekühlt wird, **dadurch gekennzeichnet, dass** im Quenchbereich (5) zunächst eine direkte Abkühlung auf eine Temperatur im Bereich von 650°C bis 1200°C durch Zuführung eines verdampfenden Quenchmediums und anschließend eine indirekte Abkühlung in einem Wärmeübertrager erfolgt.

2. Verfahren nach Anspruch 1, **dadurch gekennzeichnet, dass** man die Edukte vorvermischt.

3. Verfahren nach Anspruch 1 oder 2, **dadurch gekennzeichnet, dass** die direkte Abkühlung auf eine Temperatur im Bereich von 700°C bis 1000°C erfolgt.

4. Verfahren nach einem der Ansprüche 1 bis 3, **dadurch gekennzeichnet, dass** die indirekte Abkühlung auf weniger als 300°C erfolgt, wobei die indirekte Abkühlung vorzugsweise zur Vorheizung der Edukte oder zur Dampferzeugung genutzt wird.

5. Reaktor (1) zur Durchführung eines Verfahrens nach einem der Ansprüche 1 bis 4, **dadurch gekennzeichnet, dass** alle den Reaktionsraum (4) begrenzenden Oberflächen mit einer bei Reaktionstemperatur beständigen Feuerfestkeramik mit einem Aluminiumoxid-Anteil von mindestens 80%, bevorzugt von mindestens 95 Gew.-%, besonders bevorzugt von mindestens 96 Gew.-% ausgebildet sind, und
**dass** die Feuerfestkeramik in Form von Steinen oder Blöcken oder als Guss- oder Stampfmasse im Reaktionsraum (4) eingebracht und anschließend verdichtet, getrocknet und gebrannt wird, wobei der Brennvorgang durch die Hochtemperaturreaktion erfolgt, und wobei die Feuerfestkeramik eine Dicke im Bereich von 7 bis 30 cm aufweist.

6. Reaktor (1) nach Anspruch 5, **dadurch gekennzeichnet, dass** die Feuerfestkeramik eine Dicke von 8 bis 10 cm aufweist.

7. Reaktor (1) nach einem der Ansprüche 5 oder 6, **dadurch gekennzeichnet; dass** der Übergang des Reaktionsraumes (4) in den Quenchbereich (5) in Form eines Spaltes, der eine Breite im Bereich von 2 bis 200 mm bevorzugt im Bereich von 50 bis 150 mm aufweist, ausgebildet ist.

8. Reaktor (1) nach Anspruch 7, **dadurch gekennzeichnet, dass** die Kanäle (2) im Brennerblock (3) in Richtung der Längsachse des Reaktionsraumes (4) ausgerichtet sind, oder dass ein Teil der Kanäle (2) für das Reaktionsgemisch und/oder Kanäle (6) für die Zuführung von zusätzlichem Sauerstoff oder von Reaktionshilfsstoffen in einem beliebigen Winkel zur Längsachse des Reaktionsraumes (4) ausgerichtet sind.

9. Reaktor (1) nach einem der Ansprüche 7 oder 8, **dadurch gekennzeichnet, dass** der Quenchbereich (5) fluchtend in Richtung der Längsachse des Reaktionsraumes (4), bevorzugt als Spalt, besonders bevorzugt als Ringsspalt, ausgeführt wird.

10. Reaktor (1) nach einem der Ansprüche 5 bis 9, **dadurch gekennzeichnet, dass** die Zuführung des Quenchmediums zur direkten Abkühlung über Quenchdüsen erfolgt, die an einem oder mehreren Verteilern, bevorzugt an einem oder mehreren Ringverteilern, angebracht sind, wobei die Quenchdüsen radial oder tangential zur Hauptströmungsrichtung des Reaktionsgemisches angeordnet sind, und wobei bei mehrstufigem Zuführen bei tangentialer Anordnung eine gegenläufige Anstellung der Quenchdüsen bevorzugt ist.

11. Verwendung eines Verfahrens nach einem der Ansprüche 1 bis 4 oder eines Reaktors (1) nach einem der Ansprüche 5 bis 10 zur Herstellung von Acetylen durch partielle Oxidation von Kohlenwasserstoffen mit Sauerstoff.

## Claims

1. A process for carrying out a high-temperature reaction, in which starting materials are supplied to a reaction chamber (4) through channels (2) of a burner block (3), where in the reaction chamber (4) the high-temperature reaction having a short residence time takes place at a temperature of at least 1500°C and the reaction mixture is subsequently rapidly cooled in a quench area (5), wherein in the quench area (5) firstly a direct cooling to a temperature in the range from 650°C to 1200°C takes place by supply of an evaporating quench medium and subsequently an indirect cooling in a heat exchanger takes place.

2. The process according to claim 1, wherein the starting materials are premixed.

3. The process according to claim 1 or 2, wherein the direct cooling takes place to a temperature in the range from 700°C to 1000°C.

4. The process according to one of claims 1 to 3, wherein indirect cooling takes place to less than 300°C, the indirect cooling preferably being utilized for the preheating of the starting materials or for the generation of steam.

5. A reactor (1) for carrying out a process according to one of claims 1 to 4, wherein all the surfaces restricting the reaction chamber (4) are formed using a fire-resistant ceramic stable at reaction temperature and having an alumina content of at least 80%, preferably least 95% by weight, particular preferably at least 96% by weight, and the fire-resistant ceramic is introduced into the reaction chamber (4) in the form of stones or blocks or as a cast or tamped mass and subsequently compressed, dried and calcined, the calcining process taking place owing to the high temperature reaction, and the fire-resistant ceramic having a thickness in the range from 7 to 30 cm.

6. The reactor (1) according to claim 5, wherein the fire-resistant ceramic has a thickness of 8 to 10 cm.

7. The reactor (1) according to either of claims 5 and 6, wherein the transition of the reaction chamber (4) to the quench area (5) is designed in the form of a gap which has a width in the range from 2 to 200 mm, preferably in the range from 50 to 150 mm.

8. The reactor (1) according to claim 7, wherein the channels (2) in the burner block (3) are aligned in the direction of the longitudinal axis of the reaction chamber (4), or some of the channels (2) for the reaction chamber and/or channels (6) for the supply of additional oxygen or of reaction auxiliaries are aligned at any desired angle to the longitudinal axis of the reaction chamber (4).

9. The reactor (1) according to either of claims 7 and 8, wherein the quench area (5) is constructed aligning in the direction of the longitudinal axis of the reaction chamber (4), preferably as a gap, particularly preferably as an annular gap.

10. The reactor (1) according to one of claims 5 to 9, wherein the supply of the quench medium to the direct cooling takes place via quench nozzles which are attached to one or more distributors, preferably to one or more annular distributors, the quench nozzles being arranged radially or tangentially to the main flow direction of the reaction mixture, and where in the case of multistage supplies with tangential arrangement a countercurrent positioning of the quench nozzles is preferred.

11. The use of a process according to one of claims 1 to 4 or of a reactor (1) according to one of claims 5 to 10 for the preparation of acetylene by partial oxidation of hydrocarbons using oxygen.

## Revendications

1. Procédé d'exécution d'une réaction à haute température, au cours de laquelle des produits de départ sont amenés par des canaux (2) d'un bloc de brûleur (3) à une chambre de réaction (4), la réaction à haute température ayant lieu dans la chambre de réaction (4) avec une courte durée de séjour à une température d'au moins 1500°C et le mélange réactionnel étant ensuite rapidement refroidi dans une zone de refroidissement (5), **caractérisé en ce que** dans la zone de refroidissement (5) il se produit d'abord un refroidissement direct à une température comprise dans l'intervalle entre 650°C et 1200°C par l'amenée d'un milieu refroidissant évaporant et ensuite un refroidissement indirect dans un échangeur de chaleur.

2. Procédé selon la revendication 1, **caractérisé en ce que** les produits de départ sont préalablement mélangés.

3. Procédé selon la revendication 1 ou 2, **caractérisé en ce que** le refroidissement direct se produit à une température dans l'intervalle compris entre 700°C et 1000°C.

4. Procédé selon l'une des revendications 1 à 3, **caractérisé en ce que** le refroidissement indirect se fait à moins de 300°C, le refroidissement indirect servant de préférence au préchauffage des produits de départ ou à la production de vapeur.

5. Réacteur (1) pour l'exécution d'un procédé selon l'une des revendications 1 à 4, **caractérisé en ce que** toutes les surfaces délimitant la chambre de réaction (4) sont réalisées en une céramique réfractaire qui résiste à la température réactionnelle avec une partie en oxyde d'aluminium d'au moins 80%, de préférence d'au moins 95% en poids, de manière particulièrement préférée d'au moins 96% en poids, et
**en ce que** la céramique réfractaire est introduite sous forme de pierres ou de blocs ou comme masse coulée ou pisé réfractaire coulé dans la chambre de réaction (4), et est ensuite compactée, séchée et calcinée, le processus de calcination s'effectuant par une réaction à haute température et la céramique réfractaire présentant une épaisseur dans l'intervalle compris entre 7 et 30 cm.

6. Réacteur (1) selon la revendication 5, **caractérisé en ce que** la céramique réfractaire présente une épaisseur de 8 à 10 cm.

7. Réacteur (1) selon l'une des revendications 5 ou 6, **caractérisé en ce que** le passage de la chambre de réaction (4) à la zone de refroidissement (5) est configuré en une fente présentant une largeur dans l'intervalle compris entre 2 et 200 mm, de préférence dans l'intervalle compris entre 50 et 150 mm.

8. Réacteur (1) selon la revendication 7, **caractérisé en ce que** les canaux (2) dans le bloc de brûleur (3) sont orientés dans la direction de l'axe longitudinal de la chambre de réaction (4) ou **en ce qu'**une partie des canaux (2) pour le mélange réactionnel et/ou les canaux (6) pour l'amenée d'oxygène supplémentaire ou des adjuvants de réaction sont orientés dans un angle quelconque par rapport à l'axe longitudinal de la chambre de réaction (4).

9. Réacteur (1) selon l'une des revendications 7 ou 8, **caractérisé en ce que** la zone de refroidissement (5) est configurée en alignement dans la direction de l'axe longitudinal de la chambre de réaction (4), de préférence comme une fente, de manière particulièrement préférée comme une fente annulaire.

10. Réacteur (1) selon l'une des revendications 5 à 9, **caractérisé en ce que** l'amenée du milieu de refroidissement pour le refroidissement direct se fait par des buses de refroidissement agencées sur un ou plusieurs répartiteurs, de préférence sur un ou plusieurs répartiteurs annulaires, les buses de refroidissement étant agencées radialement ou tangentiellement à la direction du courant principal du mélange réactionnel, et on préfère, en cas d'amenée en plusieurs étapes et d'agencement tangentiel, une incidence opposée des buses de refroidissement.

11. Utilisation d'un procédé selon l'une des revendications 1 à 4, ou d'un réacteur (1) selon l'une des revendications 5 à 10 pour la préparation d'acétylène par oxydation partielle d'hydrocarbures avec de l'oxygène.
